# EUROPEAN PATENT SPECIFICATION

(11) **EP 2 886 564 B1**
(45) Date of publication and mention of the grant of the patent: **21.09.2016**
(21) Application number: 14196118.5
(22) Date of filing: 03.12.2014
(51) Int. Cl.: C08F 212/36, C08F 212/08, C08F 212/14, C08F 8/12, C07H 1/00, C07H 21/00

(54) **Porous resin bead and production method of nucleic acid by using same**
Poröse Harzperle und Herstellungsverfahren von Nukleinsäure damit
Perle de résine poreuse et procédé de production d'un acide nucléique en utilisant celui-ci

(30) Priority: 04.12.2013 JP 2013251022
(43) Date of publication of application: 24.06.2015
(73) Proprietor: Nitto Denko Corporation, Ibaraki-shi, Osaka 567-8680 (JP)
(72) Inventor: Maeta, Eri, Ibaraki-shi, Osaka 567-8680 (JP); Mori, Kenjiro, Ibaraki-shi, Osaka 567-8680 (JP); Horie, Shohei, Ibaraki-shi, Osaka 567-8680 (JP)
(74) Representative: Duckworth, Timothy John

(56) References cited:
- EP-A1- 2 055 724
- JP-A- 2009 249 478
- US-A1- 2006 051 800
- US-A1- 2009 326 210

## Description

### TECHNICAL FIELD OF THE INVENTION

The present invention relates to a porous resin bead. The porous resin bead of the present invention is useful for the production of a nucleic acid (particularly polynucleotide of not less than 40 mer).

### BACKGROUND OF THE INVENTION

Solid phase synthesis processes using a phosphoramidite method is widely applied to the chemical synthesis of nucleic acids such as DNA oligonucleotide or polynucleotide and RNA oligonucleotide or polynucleotide. In this method, for example, nucleoside to be the 3'-terminal of the nucleic acid to be synthesized is supported in advance by a solid phase synthesis support via a cleavable linker such as a succinyl group and the like, and the support is placed in a synthetic column, which is set on a nucleic acid automatic synthesizer. Thereafter, a synthesis reagent is flown in the reaction column by a nucleic acid automatic synthesizer and, for example, the following synthesis program is performed:
(1) deprotection of nucleoside 5'-OH group with trichloroacetic acid/dichloromethane solution or dichloroacetic acid/toluene solution and the like
(2) coupling reaction of amidite to the 5'-OH group by nucleoside phosphoramidite (nucleic acid monomer)/acetonitrile solution, and activator (tetrazole etc.)/acetonitrile solution
(3) capping of unreacted 5'-OH group by acetic anhydride/pyridine/methylimidazole/THF and the like
(4) oxidation of phosphite by iodine/water/pyridine and the like.

The cycle of the aforementioned synthesis program is repeated, whereby a nucleic acid having the object sequence is synthesized. The finally-synthesized nucleic acid was cut out from the solid phase synthesis support by hydrolysis of the cleavable linker with ammonia, methylamine and the like (see non-patent document 1).

As a solid phase synthesis support used for the synthesis of nucleic acid, inorganic particles such as CPG (Controlled Pore Glass) and silica gel have been used. In recent years, however, resin beads are increasingly used for large-scale synthesis at a low cost, since they can increase the synthesis amount of the nucleic acid per weight of the solid phase synthesis support. As such resin bead, low-crosslinked, highly swellable porous polystyrene bead is known (see patent document 1). The low-crosslinked, highly swellable resin bead is characterized in that many linkers to be the starting point of the nucleic acid synthesis can be bound. On the other hand, since the degree of swelling with each synthetic reagent is high, the amount of the resin beads that can be filled in a column used for the nucleic acid synthesis becomes small.

Also, improvement of the synthesis ability of nucleic acid is tried by suppressing variation in the swelling rate of a porous resin bead in various organic solvents by using acrylonitrile (see patent document 2). The porous resin bead in this document shows a small variation in the swelling rate in various organic solvents; however, further improvement in the nucleic acid synthesis ability is demanded.

In general, a longer chain length of the oligonucleotide or polynucleotide to be synthesized is problematically associated with a decrease in the synthesis ability (synthesis purity and synthesis amount). To solve this problem, the loading amount of a nucleoside linker to be the starting point of the synthesis of a solid phase synthesis support needs to be reduced. For example, when a 20 mer DNA oligonucleotide with high purity is to be synthesized, the nucleoside linker loading amount of a commercially available porous resin bead support for solid phase synthesis is about 200 µmol/g. When it is a DNA polynucleotide of not less than 40 mer, the loading amount is not more than 80 µmol/g. Consequently, the characteristic of the low-crosslinked and highly swellable resin bead, that many linkers to be the starting point of the nucleic acid synthesis can be bound thereto, cannot be utilized, and the number of the resin beads that can be filled in a column used for nucleic acid synthesis decreases, which in turn reduces the amount of the synthesized nucleic acid per single synthesis and lowers the production efficiency. In the present specification, oligonucleotide means a nucleotide polymer of 20 mer or less, and polynucleotide means a nucleotide polymer of more than 20 mer.

### [Document List]

### [patent documents]

patent document 1: JP-A-2005-325272
patent document 2: JP-A-2008-074979

### [non-patent document]

non-patent document 1: Current Protocols in Nucleic Acid Chemistry (2000) 3.6.1-3.6.13

EP2055724 provides a method for producing a porous resin bead containing an aromatic vinyl compound-hydroxystyrene-di(meth)acrylate copolymer.

### SUMMARY OF THE INVENTION

The present invention aims to provide a porous resin bead, which can be filled in a synthetic column in a large amount since it swells less with a synthetic reagent, and can improve the object product yield per the synthetic column.

The present inventors have conducted intensive studies and found that the above-mentioned object can be achieved by using a middle-crosslinked porous resin bead having a large pore size. The present invention based on the finding is as described below.
[1] A porous resin bead of a copolymer composed of a monovinyl monomer unit and a crosslinking vinyl monomer unit, which has a group capable of binding with a carboxy group by a dehydration condensation reaction,
   wherein the amount of the crosslinking vinyl monomer is 18.5 - 55 mol% of the total monomer, and
   the median pore size of the porous resin bead is 60 - 300 nm.
[2] The porous resin bead of the aforementioned [1], wherein the amount of the crosslinking vinyl monomer is 18.5 - 45 mol% of the total monomer.
[3] The porous resin bead of the aforementioned [1], wherein the amount of the crosslinking vinyl monomer is 18.5 - 40 mol% of the total monomer.
[4] The porous resin bead of the aforementioned [1], wherein the amount of the crosslinking vinyl monomer is 18.5 - 30 mol% of the total monomer.
[5] The porous resin bead of any one of the aforementioned [1] - [4], wherein the porous resin bead has a median pore size of 65 - 250 nm.
[6] The porous resin bead of any one of the aforementioned [1] - [4], wherein the porous resin bead has a median pore size of 70 - 200 nm.
[7] The porous resin bead of any one of the aforementioned [1] - [6], wherein the monovinyl monomer comprises an aromatic vinyl monomer.
[8] The porous resin bead of any one of the aforementioned [1] - [6], wherein the monovinyl monomer comprises a styrene-based monomer.
[9] The porous resin bead of any one of the aforementioned [1] - [6], wherein the monovinyl monomer is a styrene-based monomer.
[10] The porous resin bead of any one of the aforementioned [1] - [6], wherein the monovinyl monomer comprises styrene and p-hydroxystyrene.
[11] The porous resin bead of any one of the aforementioned [1] - [10], wherein the crosslinking vinyl monomer comprises a polyvalent vinyl aromatic compound.
[12] The porous resin bead of any one of the aforementioned [1] - [10], wherein the crosslinking vinyl monomer comprises divinylbenzene.
[13] The porous resin bead of any one of the aforementioned [1] - [10], wherein the crosslinking vinyl monomer comprises p-divinylbenzene and m-divinylbenzene.
[14] The porous resin bead of any one of the aforementioned [1] - [13], wherein the group capable of binding with a carboxy group by a dehydration condensation reaction is an amino group and/or a hydroxy group.
[15] The porous resin bead of any one of the aforementioned [1] - [13], wherein the group capable of binding with a carboxy group by a dehydration condensation reaction is a hydroxy group.
[16] The porous resin bead of any one of the aforementioned [1] - [15], wherein the amount of the group capable of binding with a carboxy group is 1 - 1000 µmol/g per 1 g of the porous resin bead.
[17] The porous resin bead of any one of the aforementioned [1] - [15], wherein the amount of the group capable of binding with a carboxy group is 5 - 500 µmol/g per 1 g of the porous resin bead.
[18] The porous resin bead of any one of the aforementioned [1] - [15], wherein the porous resin bead has a median particle size of 1 - 1000 µm.
[19] The porous resin bead of any one of the aforementioned [1] - [15], wherein the porous resin bead has a median particle size of 10 - 500 µm.
[20] The porous resin bead of any one of the aforementioned [1] - [15], wherein the porous resin bead has a median particle size of 20 - 300 µm.
[21] A method of producing a nucleic acid, comprising sequentially binding a nucleoside or a nucleotide with the porous resin bead of any one of the aforementioned [1] - [20] via a cleavable linker to give an oligonucleotide or a polynucleotide.
[22] The production method of [21], wherein the loading amount of the cleavable linker is 1 - 80 µmol/g per 1 g of the porous resin bead.
[23] The production method of [21], wherein the loading amount of the cleavable linker is 5 - 80 µmol/g per 1 g of the porous resin bead.
[24] The production method of [21], wherein the loading amount of the cleavable linker is 10 - 80 µmol/g per 1 g of the porous resin bead.
[25] The production method of any one of [21] - [24], which produces a polynucleotide of not less than 40 mer.

### Effect of the Invention

Since the porous resin bead of the present invention swells less in a synthetic reagent and is filled in a large amount in a synthetic column, it can increase the yield of the object product per the synthetic column. Using the porous resin bead of the present invention, therefore, a nucleic acid (particularly, DNA polynucleotide having a long base sequence) can be synthesized efficiently.

### DETAILED DESCRIPTION OF THE INVENTION

The porous resin bead of the present invention is a copolymer composed of a monovinyl monomer unit and a crosslinking vinyl monomer unit. The monovinyl monomer is not particularly limited as long as it is a monomer having one vinyl group. Examples of the monovinyl monomer include aromatic vinyl monomer, alkyl (meth)acrylate, vinyl acetate, (meth)acrylonitrile, vinylpyridine, vinylpyrrolidone and the like. Only one kind of a monovinyl monomer may be used or two or more kinds thereof may be used in combination. Preferred as the monovinyl monomer is an aromatic vinyl monomer or alkyl (meth)acrylate, and more preferred is an aromatic vinyl monomer.

Examples of the aromatic vinyl monomer include a 5- or 6-membered aromatic ring compound having one vinyl group, and optionally having a hetero atom such as a nitrogen atom and the like as an annular atom. The aromatic ring optionally has one or more substituents such as a methyl group, an acetoxy group and the like. Preferred as the aromatic vinyl monomer is a styrene-based monomer.

Examples of the styrene-based monomer include styrene; alkylstyrene such as ethylstyrene, methylstyrene, dimethylstyrene, trimethylstyrene, butylstyrene and the like; halogenated styrene such as chlorostyrene, dichlorostyrene, fluorostyrene, pentafluorostyrene, bromostyrene and the like; halogenated alkylstyrene such as chloromethylstyrene, fluoromethylstyrene and the like; aminostyrene; cyanostyrene; alkoxystyrene such as methoxystyrene, ethoxystyrene, butoxy styrene and the like; acyloxystyrene such as acetoxystyrene and the like; nitrostyrene; and the like. In styrene (e.g., alkylstyrene) having a substituent (e.g., an alkyl group), the position of a substituent may be any of the ortho-position, meta-position and para-position, and the para-position is preferable.

Examples of the alkyl (meth)acrylate include an ester obtained from straight chain or branched chain monohydric alcohol, wherein the carbon number of the alkyl group is 1 - 10, and (meth)acrylic acid, and the like. Concrete examples thereof include methyl acrylate, butyl acrylate, 2-ethylhexyl acrylate, methoxyethyl acrylate, methoxyethylene glycol acrylate, methoxypolyethylene glycol acrylate, methyl methacrylate, ethyl methacrylate, butyl methacrylate, hexyl methacrylate, 2-ethylhexyl methacrylate, glycidyl methacrylate, stearyl methacrylate, 2-hydroxyethyl methacrylate, methoxyethylene glycol methacrylate, methoxypolyethylene glycol methacrylate, polyethylene glycol methacrylate, benzyl methacrylate, trifluoroethyl methacrylate, octafluoropentyl methacrylate and the like.

A crosslinking vinyl monomer is used as a crosslinking agent, and is not particularly limited as long as it has two or more vinyl groups. The number of the vinyl groups in the crosslinking vinyl monomer is preferably 2 - 3. Examples of the crosslinking vinyl monomer include polyvalent vinyl aromatic compounds such as divinylbenzene (o-divinylbenzene, m-divinylbenzene, p-divinylbenzene), trivinylbenzene (e.g., 1,3,5-trivinylbenzene) and the like; trivinylcyclohexane; di(meth)acrylate compounds such as ethylene glycol di(meth)acrylate, diethylene glycol di(meth)acrylate, triethylene glycol di(meth)acrylate, polyethylene glycol di(meth)acrylate, propylene glycol di(meth)acrylate, dipropylene ethylene glycol di(meth)acrylate, tripropylene glycol di(meth)acrylate, polypropylene glycol di(meth)acrylate, butanediol di(meth)acrylate, hexanediol di(meth)acrylate, nonanediol di(meth)acrylate and the like; and the like. Only one kind of a crosslinking vinyl monomer may be used or two or more kinds thereof may be used in combination. Preferred as the crosslinking vinyl monomer is a polyvalent vinyl aromatic compound, more preferred is divinylbenzene, and further preferred are p-divinylbenzene, m-divinylbenzene, and a mixture of p-divinylbenzene and m-divinylbenzene.

The porous resin bead of the present invention has a group capable of binding with a carboxy group by a dehydration condensation reaction. When a nucleic acid is synthesized, nucleoside is linked with a porous resin bead. In this case, when the linker has a carboxy group, the carboxy group of the linker and the porous resin bead can be bound with ease. Examples of the group capable of binding with a carboxy group include an amino group (primary amino group, secondary amino group) and a hydroxy group, with preference given to a hydroxy group.

A method of introducing a group capable of binding with a carboxy group into the porous resin bead of the present invention is not particularly limited. For example, when the group capable of binding with a carboxy group is a hydroxy group, for example, 2-hydroxyethyl acrylate, 2-hydroxyethyl methacrylate, 2-hydroxypropyl acrylate, 2-hydroxypropyl methacrylate, 4-hydroxybutyl acrylate, 4-hydroxybutyl methacrylate, hydroxystyrene and the like are copolymerized as monovinyl monomers. When the group capable of binding with a carboxy group is an amino group, for example, aminostyrene, aminomethylstyrene and the like are copolymerized as monovinyl monomers.

The porous resin bead having a group capable of binding with a carboxy group by a dehydration condensation reaction may be produced by producing a porous resin bead having other functional group, and converting the functional group to a group capable of binding with a carboxy group.

A porous resin bead having other functional group can be produced by copolymerizing, for example, acylaminostyrene such as acetylaminostyrene and the like; acyloxystyrene such as acetoxystyrene, ethanoyloxystyrene, benzoyloxystyrene and the like; and haloalkylstyrene such as chloromethylstyrene and the like, as monovinyl monomers.

A porous resin bead having an acyloxy group or acylamino group as other functional groups can be converted, by hydrolysis, to a porous resin bead having a hydroxy group or amino group. A porous resin bead having a haloalkyl group as other functional group can be converted to a porous resin bead having an amino group or hydroxy group by a reaction with phthalimide and hydrazine, ammonia or sodium hydroxide, and the like.

The amount of a group capable of binding with a carboxy group is preferably 1 - 1000 µmol/g, more preferably 5 - 500 µmol/g, per 1 g of the porous resin bead. When the amount is less than 1 µmol/g, the amount of synthesized nucleic acid becomes small since the loading amount of a cleavable linker to be the starting point of synthesis becomes small when the bead is used as a support for the solid phase synthesis. When the amount exceeds 1000 µmol/g, the loading of the cleavable linker in the porous resin bead is biased and, when the distance between the adjacent cleavable linkers is insufficient, the chemical reactions that occur in adjacency are inhibited by each other. Consequently, when the bead is used as a support for the solid phase synthesis, the obtained nucleic acid tends to have low purity.

In the porous resin bead of the present invention, the amount of the crosslinking vinyl monomer is 18.5 - 55 mol%, preferably 18.5 - 45 mol%, more preferably 18.5 - 40 mol%, further preferably 18.5 - 30 mol%, of the total monomer. When the amount is less than 18.5 mol%, the obtained bead swells more in a synthetic reagent. When the amount exceeds 55 mol%, the amount of the synthesized nucleic acid decreases since the loading efficiency of a cleavable linker decreases and the loading amount thereof becomes small.

The median pore size of the porous resin bead of the present invention is 60 - 300 nm, preferably 65 - 250 nm, more preferably 70 - 200 nm. When this median pore size is less than 60 nm, the purity of the nucleic acid tends to be low. When the median pore size exceeds 300 nm, the porosity of the porous resin bead becomes high and the dry volume becomes large. As a result, the amount of the beads to be filled in a column used for the synthesis becomes small, the amount of the synthesized nucleic acid per single synthesis becomes small, and the production efficiency decreases. The median pore size is a value measured by a mercury penetration method. To be specific, 0.2 g of porous resin beads are cast in a mercury porosimeter PoreMaster60-GT (manufactured by QuantaChrome), and the median pore size is measured by a mercury penetration method under the conditions of mercury contact angle 140° and mercury surface tension 480 dyn/cm.

The shape of the porous resin bead of the present invention is not necessarily an exact spherical shape, but at least a granular shape. However, the porous resin bead of the present invention is preferably spherical since it can enhance the filling efficiency into a reaction column for solid phase synthesis and resists breakage.

While the median particle size of the porous resin bead of the present invention is not particularly limited, it is preferably 1 - 1000 µm, more preferably 10 - 500 µm, further preferably 20 - 300 µm. The median particle size is a value measured by a laser diffraction scattering method. To be specific, the median particle size can be measured by a laser diffraction scattering particle size distribution analyzer LA-950 (manufactured by Horiba, Ltd.) and using a 50 v/v% aqueous ethanol solution as a dispersing medium.

When a porous resin bead is produced by suspension polymerization, the median particle size of the porous resin bead can be controlled to fall within a desirable range by adjusting the stirring condition before the start of polymerization and the concentration of a dispersion stabilizer.

The production method of the porous resin bead of the present invention is not particularly limited and examples thereof include
(1) a method including stirring a monovinyl monomer, a crosslinking vinyl monomer, a vinyl monomer having a group capable of binding with a carboxy group by a dehydration condensation reaction, a pore-forming agent and a polymerization initiator in water containing a dispersion stabilizer dispersed or dissolved therein, and conducting suspension copolymerization,
(2) a method including stirring a monovinyl monomer, a crosslinking vinyl monomer, a vinyl monomer having other functional group, a pore-forming agent and a polymerization initiator in water containing a dispersion stabilizer dispersed or dissolved therein, conducting suspension copolymerization to give a porous resin bead having other functional group, and converting, by hydrolysis and the like, said other functional group to a group capable of binding with a carboxy group by a dehydration condensation reaction, and the like.

The amount of the vinyl monomer containing a group capable of binding with a carboxy group by a dehydration condensation reaction or having the aforementioned other functional group is preferably 1 - 15 mol%, more preferably 1 - 10 mol%, of the total monomer.

The suspension copolymerization is performed by emulsifying a mixed solution of the aforementioned respective monomers, pore-forming agent and polymerization initiator by stirring same in water containing a dispersion stabilizer dispersed or dissolved therein.

The aforementioned pore-forming agent means a solvent other than water in a suspension copolymerization system, and is preferably hydrocarbon or alcohol. The hydrocarbon is a saturated or unsaturated aliphatic hydrocarbon or aromatic hydrocarbon, preferably an aliphatic hydrocarbon having 5 - 12 carbon atoms, more preferably n-hexane, n-heptane, n-octane, isooctane, undecane, dodecane or the like. To increase the porosity of the obtained porous resin bead, hydrocarbon and alcohol are desirably used in combination. Examples of alcohol include aliphatic alcohol preferably having 5 - 9 carbon atoms. Specific examples of such alcohol include 2-ethylhexanol, t-amylalcohol, nonylalcohol, 2-octanol, cyclohexanol and the like.

The weight ratio of the hydrocarbon and alcohol to be used as a pore-forming agent is appropriately changed depending on the specific combination thereof, based on which a specific surface area of the support for solid phase synthesis obtained thereby can be increased. A preferable weight ratio of the hydrocarbon and alcohol is 1:9 - 6:4.

The weight of the pore-forming agent in suspension copolymerization is preferably 0.5- to 2.5-fold, more preferably 0.8- to 2.3-fold, further preferably 1.0- to 2.2-fold, of the total weight of the above-mentioned respective monomers. When the weight is outside the range of 0.5- to 2.5-fold, the obtained porous resin bead has a smaller pore size and a smaller specific surface area, and the amount of the reaction product synthesized by the chemical reaction becomes small.

The method of suspension copolymerization is not particularly limited and a known method can be used.

The dispersion stabilizer is not particularly limited, and known hydrophilic protective colloid agents such as polyvinyl alcohol, polyacrylic acid, gelatin, starch, carboxymethylcellulose and the like; poorly soluble powders such as calcium carbonate, magnesium carbonate, calcium phosphate, barium sulfate, calcium sulfate, bentonite and the like; and the like are used. The amount of the dispersion stabilizer to be added is not particularly limited, and is preferably 0.01 - 10 parts by weight relative to 100 parts by weight of water in the suspension polymerization system. When the amount of the dispersion stabilizer is small, the dispersion stability of the suspension polymerization is impaired and many aggregates are formed. When the amount of the dispersion stabilizer is high, many fine beads are formed.

The polymerization initiator is not particularly limited, and known peroxides such as dibenzoyl peroxide, dilauroyl peroxide, distearoyl peroxide, 1,1-di(t-butylperoxy)-2-methylcyclohexane, 1,1-di(t-hexylperoxy)-3,3,5-trimethylcyclohexane, 1,1-di(t-hexylperoxy)cyclohexane, 1,1-di(t-butylperoxy)cyclohexane, di-t-hexyl peroxide, t-butylcumyl peroxide, di-t-butyl peroxide, 1,1,3,3-tetramethylbutylperoxy-2-ethyl hexanoate, t-hexylperoxy-2-ethyl hexanoate, t-butylperoxy-2-ethyl hexanoate, t-butylperoxyisopropyl monocarbonate and the like; azo compounds such as 2,2'-azobisisobutyronitrile, 2,2'-azobis-2-methylbutyronitrile, 2,2'-azobis-2,4-dimethylvaleronitrile and the like are used. The amount of the polymerization initiator to be added is not particularly limited, and those of ordinary skill in the art can determine an appropriate amount thereof.

The reaction conditions of the suspension copolymerization can be determined appropriately. The temperature of the suspension copolymerization is, for example, 60 - 90°C, and the time thereof is, for example, 30 min - 48 hr. The suspension copolymerization is generally performed with stirring. The stirring rate is, for example, 100 rpm - 1000 rpm, preferably 200 rpm - 800 rpm.

The porous resin bead obtained by the suspension copolymerization may be subjected to an appropriate treated treatment such as washing, drying, classification and the like.

The porous resin bead of the present invention can be obtained by the above-mentioned processing. The porous resin bead can be utilized as a solid phase support for chemical synthesis. The porous resin bead of the present invention can be particularly used effectively as a solid phase support for nucleic acid synthesis.

A conventionally-known method can be applied to nucleic acid synthesis using the porous resin bead of the present invention. For example, the following nucleoside succinyl linker is bound as a cleavable linker to the porous resin bead (solid phase support) of the present invention.

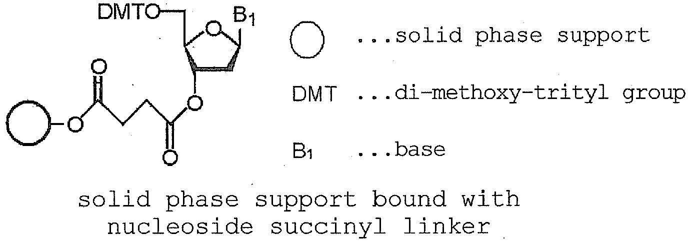

Examples of the nucleoside succinyl linker include, in addition to the above, a linker corresponding to RNA or modified nucleotide, a universal linker and the like.

Then, nucleoside phosphoramidite is bound one by one such that a predetermined base sequence is obtained from the 5'-terminal of nucleoside. This synthesis reaction can be performed by using an automatic synthesizer. For example, a 5'-OH deprotecting agent solution, a nucleoside phosphoramidite solution, an amidite activator solution, an oxidant solution, a capping agent solution, acetonitrile as a washing solution and the like are successively delivered to a reaction column of the apparatus filled with porous resin beads bound with a nucleoside succinyl linker, and the reaction is repeated. Finally, the succinyl linker moiety is cleaved by hydrolysis with an alkali solution, and the like, whereby the object nucleic acid can be obtained.

In the production method of the nucleic acid of the present invention, the loading amount of the cleavable linker is preferably 1 - 80 µmol/g, more preferably 5 - 80 µmol/g, further preferably 10 - 80 µmol/g, per 1 g of the porous resin bead. When the loading amount of the cleavable linker is less than 1 µmol/g, the amount of the nucleic acid to be synthesized decreases. When the loading amount of the cleavable linker exceeds 80 µmol/g, the loading of the cleavable linker in the porous resin bead is biased and, when the distance between the adjacent cleavable linkers is insufficient, the chemical reactions that occur in adjacency are inhibited by each other. Consequently, when the group is used as a support for the solid phase synthesis, the obtained nucleic acid tends to have low purity.

A production method of a nucleic acid by using the porous resin bead of the present invention is particularly useful for the synthesis of a polynucleotide of not less than 40 mer.

### Examples

The present invention is concretely explained in more detail by referring to the following Examples and Comparative Examples.

### Example 1

### (1) Production of porous resin bead

A 500 mL separable flask with a cooler, a stirrer and a nitrogen inlet tube was set on a thermostatic water bath, polyvinyl alcohol (manufactured by KURARAY CO., LTD., 2.6 g) and distilled water (260 g) were added, and the mixture was stirred at 300 rpm to dissolve polyvinyl alcohol. Thereto was added a solution obtained by mixing styrene (manufactured by Wako Pure Chemical Industries, Ltd., 34.36 g, 71.6 mol% in total monomer), p-acetoxystyrene (manufactured by Aldrich, 3.82 g, 5.1 mol% in total monomer), divinylbenzene (mixture of m-divinylbenzene and p-divinylbenzene, content 55 wt%, manufactured by Wako Pure Chemical Industries, Ltd., 25.45 g, 23.3 mol% in total monomer), 2-ethylhexanol (manufactured by Wako Pure Chemical Industries, Ltd., 53.45 g), isooctane (manufactured by Wako Pure Chemical Industries, Ltd., 22.91 g) and benzoyl peroxide (containing 25 wt% water, manufactured by NOF CORPORATION, 1.27 g). The mixture was stirred (500 rpm) under a nitrogen stream at room temperature and heated to 80°C, and suspension copolymerization was performed for 10 hr.

The polymerization product was washed by filtration with distilled water and acetone (manufactured by Wako Pure Chemical Industries, Ltd.), and dispersed in acetone to the total amount of about 1 L. The dispersion was left standing until the precipitate became undisturbed even when the dispersion was tilted, and acetone in the supernatant was discarded. Acetone was added again to the precipitate to the total amount of about 1 L, and the mixture was classified by repeating the operation of standing still and acetone discarding. The dispersion was filtered and dried under reduced pressure to give porous resin beads of a styrene-divinylbenzene-p-acetoxystyrene copolymer.

Then, into a 500 mL flask equipped with a cooler, a stirrer and a nitrogen inlet tube were charged the above-mentioned porous resin beads (20 g), ethanol (80 g), distilled water (20 g) and sodium hydroxide (0.8 g), and the mixture was reacted for 5 hr by stirring at 80.5°C. The dispersion was neutralized with hydrochloric acid, washed by filtration with distilled water and acetone, and dried under reduced pressure to give porous resin beads of a styrene-divinylbenzene-p-hydroxystyrene copolymer (amount of hydroxyl group per 1 g of porous resin beads as calculated from the monomer amount: 459 µmol/g).

### (2) Measurement of median pore size

The obtained porous resin beads (0.2 g) were cast into a mercury porosimeter PoreMaster60-GT (manufactured by QuantaChrome), and the median pore size of the porous resin beads was measured by a mercury penetration method under the conditions of mercury contact angle 140° and mercury surface tension 480 dyn/cm. The results are shown in Table 1.

### (3) Measurement of median particle size

The obtained porous resin bead was dispersed by ultrasonication in 50 v/v% aqueous ethanol solution. The dispersion was used as a sample, and the median particle size of the porous resin bead was measured by a laser diffraction/scattering type particle size distribution measuring apparatus LA-920 (manufactured by Horiba, Ltd.) using 50 v/v% aqueous ethanol solution as a dispersing medium. The results are shown in Table 1.

### (4) Measurement of swelling volume in toluene

The obtained porous resin beads (1 g) were weighed and cast into a 10 mL measuring cylinder. Thereafter, toluene was added, the mixture was left standing overnight, and the swelling volume was read on the scale of the measuring cylinder. The swelling volume and the maximum amount of the beads to be filled in a 0.2 mL column, which is calculated from said volume, are shown in Table 1.

### (5) Loading of DMT-dT-3'-succinate to porous resin bead

According to the formulation shown in Table 2, the porous resin bead obtained in Example 1, DMT-dT-3'-succinate (manufactured by Beijing OM Chemicals), O-(benzotriazol-1-yl)-N,N,N',N'-tetramethyluronium hexafluorophosphate (HBTU, manufactured by Novabiochem), N,N-diisopropylethylamine (DIPEA, manufactured by Aldrich) and acetonitrile (manufactured by Wako Pure Chemical Industries, Ltd.) were mixed and reacted by stirring at room temperature for 12 hr. Then, after washing by filtration with acetonitrile, the obtained porous resin beads were dried under reduced pressure.

The porous resin beads dried under reduced pressure were mixed with CapA (20 wt% acetic anhydride/80 wt% acetonitrile, 112.5 mL), CapB (20 wt% N-methylimidazole/30 wt% pyridine/50 wt% acetonitrile, 12.5 mL), 4-dimethylaminopyridine (manufactured by Aldrich, 63 mg) and acetonitrile (12.5 mL), and the mixture was reacted by stirring at room temperature for 12 hr, washed by filtration with acetonitrile, and dried under reduced pressure to give porous resin beads bound with DMT-dT-3'-succinate. The loading amount of DMT-dT-3'-succinate per 1 g of the porous resin beads was determined by the measurement of the absorbance at 412 nm of the DMT group deprotected with p-toluenesulfonic acid/acetonitrile solution. The loading amount is shown in Table 2.

### (6) Synthesis of 60 mer DNA polynucleotide

The obtained porous resin beads bound with DMT-dT-3'-succinate were placed in a synthetic column (volume 0.2 mL) to a synthesis scale of 1 µmol, which was set on ABI3400 DNA/RNA synthesizer (manufactured by Applied Biosystems), and DNA polynucleotide of 60 mer mixed sequence was synthesized under the conditions of nucleoside phosphoramidite concentration 4 eq/synthesis scale, DMT-on. After the synthesis, DNA polynucleotide was cut out from the dried porous resin beads and the base amino group was deprotected. The OD yield (corresponding to nucleic acid synthesis amount) of the nucleic acid was determined from the UV absorbance measurement (measurement wavelength: 260 nm) of a filtrate after separation of the porous resin beads by filter filtration. Then, the filtrate was subjected to HPLC measurement (HPLC apparatus manufactured by Waters Corporation was used), and the synthesis purity (full-length (area%)), and proportion of DNA polynucleotide having the object sequence length) was determined. The results are shown in Table 3.

### Example 2

In the same manner as in Example 1 except that styrene (20.16 g, 44.8 mol% in total monomer), p-acetoxystyrene (3.82 g, 5.5 mol% in total monomer), divinylbenzene (content 55 wt%, 50.9 g, 49.8 mol% in total monomer), 2-ethylhexanol (55.39 g), isooctane (23.74 g) and benzoyl peroxide (1.22 g) were used, porous resin beads of a styrene-divinylbenzene-p-hydroxystyrene copolymer were obtained (amount of hydroxyl group per 1 g of porous resin bead as calculated from the monomer amount: 466 µmol/g).

The median pore size, the median particle size, the swelling volume in toluene and the maximum bead filling amount of the porous resin beads in a 0.2 mL column, which were measured and calculated in the same manner as in Example 1, are shown in Table 1.

In the same manner as in Example 1 and using the blending ratio shown in Table 2, porous resin beads bound with DMT-dT-3'-succinate were produced, and the DMT-dT-3'-succinate loading amount per 1 g of the porous resin beads was measured. The loading amount is shown in Table 2.

In the same manner as in Example 1, a DNA polynucleotide of a 60 mer mixed sequence was synthesized, and the OD yield and full-length (area%) of the nucleic acid were determined. The results are shown in Table 3.

### Comparative Example 1

In the same manner as in Example 1 except that styrene (45.02 g, 85.4 mol% in total monomer), p-acetoxystyrene (3.65 g, 4.4 mol% in total monomer), divinylbenzene (content 55 wt%, 12.2 g, 10.2 mol% in total monomer), 2-ethylhexanol (55.39 g), isooctane (23.74 g) and benzoyl peroxide (1.22 g) were used, porous resin beads of a styrene-divinylbenzene-p-hydroxystyrene copolymer were obtained (amount of hydroxyl group per 1 g of the porous resin beads as calculated from the monomer amount: 409 µmol/g).

The median pore size, the median particle size, the swelling volume in toluene and the maximum bead filling amount of the porous resin beads in a 0.2 mL column, which were measured and calculated in the same manner as in Example 1, are shown in Table 1.

In the same manner as in Example 1 and using the blending ratio shown in Table 2, porous resin beads bound with DMT-dT-3'-succinate were produced, and the DMT-dT-3'-succinate loading amount per 1 g of the porous resin beads was measured. The loading amount is shown in Table 2.

In the same manner as in Example 1, a DNA polynucleotide of a 60 mer mixed sequence was synthesized, and the OD yield and full-length (area%) of the nucleic acid were determined. The results are shown in Table 3.

### Comparative Example 2

In the same manner as in Example 1 except that p-acetoxystyrene (3.2 g, 8.4 mol% in total monomer), divinylbenzene (content 55 wt%, 50.62 g, 91.6 mol% in total monomer), 2-ethylhexanol (60.31 g) and isooctane (25.85 g) were used, porous resin beads of a divinylbenzene-p-hydroxystyrene copolymer were obtained (amount of hydroxyl group per 1 g of the porous resin beads as calculated from the monomer amount: 649 µmol/g).

The median pore size, the median particle size, the swelling volume in toluene and the maximum bead filling amount of the porous resin beads in a 0.2 mL column, which were measured and calculated in the same manner as in Example 1, are shown in Table 1.

In the same manner as in Example 1 and using the blending ratio shown in Table 2, porous resin beads bound with DMT-dT-3'-succinate were produced, and the DMT-dT-3'-succinate loading amount per 1 g of the porous resin beads was measured. The loading amount is shown in Table 2.

In the same manner as in Example 1, a DNA polynucleotide of a 60 mer mixed sequence was synthesized, and the OD yield and full-length (area%) of the nucleic acid were determined. The results are shown in Table 3.

### Comparative Example 3

In the same manner as in Example 1 except that styrene (32.27 g, 61.5 mol% in total monomer), p-acetoxystyrene (3.9 g, 4.8 mol% in total monomer), divinylbenzene (content 55 wt%, 11.1 g, 9.3 mol% in total monomer), methacrylonitrile (8.24 g, 24.4 mol% in total monomer), 2-ethylhexanol (59.57 g), isooctane (25.53 g) and benzoyl peroxide (1.10 g) were used, porous resin beads of a styrene-divinylbenzene-p-hydroxystyrene-methacrylonitrile copolymer were obtained (amount of hydroxyl group per 1 g of porous resin bead as calculated from the monomer amount: 488 µmol/g).

The median pore size, the median particle size, the swelling volume in toluene and the maximum bead filling amount of the porous resin beads in a 0.2 mL column, which were measured and calculated in the same manner as in Example 1, are shown in Table 1.

In the same manner as in Example 1 and using the blending ratio shown in Table 2, porous resin beads bound with DMT-dT-3'-succinate were produced, and the loading amount of DMT-dT-3'-succinate per 1 g of the porous resin beads was measured. The loading amount is shown in Table 2.

In the same manner as in Example 1, a DNA polynucleotide of a 60 mer mixed sequence was synthesized, and the OD yield and full-length (area%) of the nucleic acid were determined. The results are shown in Table 3.

**Table 1**

| | amount of crosslinking vinyl monomer (divinylbenzene) in total monomer (mol%) | median pore size of bead (nm) | median particle size of bead (µm) | swelling volume of bead in toluene (mL/g) | maximum bead filling amount in 0.2 mL column (mg) |
|---|---|---|---|---|---|
| Ex. 1 | 23.3 | 71 | 100 | 5.4 | 37.0 |
| Ex. 2 | 44.8 | 118 | 96 | 6.0 | 33.3 |
| Comp. Ex. 1 | 10.2 | 66 | 94 | 6.6 | 30.3 |
| Comp. Ex. 2 | 91.6 | 148 | 93 | 6.0 | 33.3 |
| Comp. Ex. 3 | 9.3 | 47 | 87 | 6.1 | 32.8 |

**Table 2**

| | bead (g) | DMT-dT-3'-succinate (g) | HBTU (g) | acetonitrile (mL) | DIPEA (µL) | DMT-dT-3'-succinate loading amount (µmol/g) |
|---|---|---|---|---|---|---|
| Ex. 1 | 3.0 | 0.247 | 0.126 | 30 | 0.114 | 85.5 |
| Ex. 2 | 3.0 | 0.287 | 0.151 | 30 | 0.136 | 83.0 |
| Comp. Ex. 1 | 5.0 | 0.328 | 0.167 | 50 | 0.153 | 86.9 |
| Comp. Ex. 2 | 3.0 | 0.344 | 0.175 | 30 | 0.158 | 82.0 |
| Comp. Ex. 3 | 5.0 | 0.328 | 0.167 | 50 | 0.153 | 82.7 |

**Table 3**

| | OD yield (OD/ µmol) | full-length (area%) | calculated maximum nucleic acid synthesis scale (µmol) of 0.2 mL column | calculated yield of object product per 0.2 mL column |
|---|---|---|---|---|
| Ex. 1 | 194 | 55.0 | 3.16 | 337 |
| Ex. 2 | 197 | 52.0 | 2.76 | 283 |
| Comp. Ex. 1 | 179 | 58.7 | 2.63 | 276 |
| Comp. Ex. 2 | 223 | 45.4 | 2.73 | 276 |
| Comp. Ex. 3 | 183 | 54.0 | 2.70 | 267 |

Table 3 also describes the calculated maximum nucleic acid synthesis scale (µmol) of 0.2 mL column (= column volume (0.2 mL) x DMT-dT-3' succinate loading amount (µmol/g)/swelling volume in toluene (mL/g)), and the calculated yield of object product per 0.2 mL column (=maximum nucleic acid synthesis scale (µmol) in 0.2 mL column x OD yield (OD/ µmol) x full-length (area%)/100) as calculated using the above-mentioned value. As shown in Table 3, the porous resin beads of Examples 1 and 2 (particularly, Example 1) of the present invention, which satisfy the requirements of the crosslinking monomer amount and median pore size, show a higher calculated yield of the object product per a 0.2 mL column than the porous resin beads of Comparative Examples 1 - 3, which fail to satisfy the requirements of the present invention. The results reveal that a nucleic acid (particularly, DNA polynucleotide with a long base sequence) can be synthesized efficiently by using the porous resin bead of the present invention.

### INDUSTRIAL APPLICABILITY

The present invention provides a porous resin bead useful as a support for solid phase synthesis of nucleic acid. The porous resin bead of the present invention can efficiently synthesize a nucleic acid (particularly, DNA polynucleotide having a long base sequence).

## Claims

1. A porous resin bead of a copolymer composed of a monovinyl monomer unit and a crosslinking vinyl monomer unit, which has a group capable of binding with a carboxy group by a dehydration condensation reaction,
wherein the amount of the crosslinking vinyl monomer is 18.5 - 55 mol% of the total monomer, and
the median pore size of the porous resin bead is 60 - 300 nm.

2. The porous resin bead according to claim 1, wherein the monovinyl monomer comprises a styrene-based monomer.

3. The porous resin bead according to claim 1 or 2, wherein the group capable of binding with a carboxy group by a dehydration condensation reaction is an amino group and/or a hydroxy group.

4. A method of producing a nucleic acid, comprising sequentially binding a nucleoside or a nucleotide with the porous resin bead according to any one of claims 1 to 3 via a cleavable linker to give an oligonucleotide or a polynucleotide.

5. The production method according to claim 4, which produces a polynucleotide of not less than 40 mer.

## Patentansprüche

1. Poröse Harzperle aus einem Copolymer, das aus einer Monovinyl-Monomereinheit und einer vernetzenden Vinyl-Monomereinheit besteht, wobei die Harzperle eine Gruppe aufweist, die durch eine Dehydratisierungskondensationsreaktion an eine Carboxygruppe binden kann;
wobei die Menge des vernetzenden Vinylmonomers 18,5 bis 55 Mol-% des gesamten Monomers beträgt; und
der Medianwert der Porengröße der porösen Harzperle 60 bis 300 nm beträgt.

2. Poröse Harzperle gemäß Anspruch 1, wobei das Monovinylmonomer ein Monomer auf Styrolbasis umfasst.

3. Poröse Harzperle gemäß Anspruch 1 oder 2, wobei die Gruppe, die durch eine Dehydratisierungskondensationsreaktion an eine Carboxygruppe binden kann, eine Aminogruppe und/oder eine Hydroxygruppe ist.

4. Verfahren zur Herstellung einer Nucleinsäure, umfassend die sukzessive Bindung eines Nucleosids oder eines Nucleotids an die poröse Harzperle gemäß einem der Ansprüche 1 bis 3 über einen abspaltbaren Linker, was ein Oligonucleotid oder ein Polynucleotid ergibt.

5. Herstellungsverfahren gemäß Anspruch 4, das ein Polynucleotid von nicht weniger als 40 mer erzeugt.

## Revendications

1. Perle de résine poreuse d'un copolymère composé d'une unité monomère monovinylique et d'une unité monomère vinylique de réticulation, qui comporte un groupe capable de se lier à un groupe carboxy par une réaction de condensation - déshydratation,
dans laquelle la quantité du monomère vinylique de réticulation est de 18,5 à 55 % en mol du monomère total, et
la taille de pore médiane de la perle de résine poreuse est de 60 à 300 nm.

2. Perle de résine poreuse selon la revendication 1, dans laquelle le monomère vinylique comprend un monomère à base de styrène.

3. Perle de résine poreuse selon la revendication 1 ou 2, dans laquelle le groupe capable de se lier à un groupe carboxy par une réaction de condensation - déshydratation est un groupe amino et/ou un groupe hydroxy.

4. Procédé de production d'un acide nucléique, comprenant la liaison séquentielle d'un nucléoside ou d'un nucléotide à la perle de résine poreuse selon l'une quelconque des revendications 1 à 3 via un agent de liaison clivable pour donner un oligonucléotide ou un polynucléotide.

5. Procédé de production selon la revendication 4, qui produit un polynucléotide de pas moins de 40 mer.
